# EUROPÄISCHE PATENTSCHRIFT

(11) **EP 0 904 117 B1**
(45) Veröffentlichungstag und Bekanntmachung des Hinweises auf die Patenterteilung: **12.07.2000**
(21) Anmeldenummer: 97916428.2
(22) Anmeldetag: 02.04.1997
(51) Int. Cl.: A61M 1/10

(54) **INTRAVASALE BLUTPUMPE**
INTRAVASCULAR BLOOD PUMP
POMPE A SANG INTRAVASCULAIRE

(30) Priorität: 04.04.1996 DE 19613564
(43) Veröffentlichungstag der Anmeldung: 31.03.1999
(73) Patentinhaber: Impella Cardiotechnik Aktiengesellschaft, 52074 Aachen (DE)
(72) Erfinder: Rau, Gunter, 52068 Aachen (DE); Reul, Helmut, 52353 Düren (DE); Siess, Thorsten, 52072 Aachen (DE)
(74) Vertreter: Selting, Günther, Dipl.-Ing.
(86) Internationale Anmeldenummer: EP9701661
(87) Internationale Veröffentlichungsnummer: WO9737698

(56) Entgegenhaltungen:
- EP-A- 0 560 466
- EP-A- 0 611 580
- WO-A-94/09835
- US-A- 5 211 546
- US-A- 5 503 615

## Beschreibung

Die Erfindung betrifft eine intravasale Blutpumpe mit einem Antriebsteil und einem Pumpenteil, der durch das Gefäßsystem des menschlichen Körpers hindurchgeführt werden kann, um beispielsweise im Herzen Pumparbeit auszuführen.

Eine intravasale Blutpumpe wird durch Punktion eines Blutgefäßes in das Gefäßsystem des Körpers eingeführt und zum Herzen oder an eine andere Stelle, an der Blut gepumpt werden soll, vorgeschoben. Diejenigen Teile, die in den Körper eingeführt werden, müssen im Durchmesser so klein sein, daß sie durch die extern zugänglichen, großen Gefäße hindurchpassen. Der größte zulässige Durchmesser liegt bei etwa 7 mm.

Aus EP 0 157 871 B1 und EP 0 397 668 B1 sind intravasale Blutpumpen bekannt, bei denen der Pumpenteil ein rohrförmiges Gehäuse enthält, in dem ein Flügelrad drehbar angeordnet ist. Das Flügelrad ist über eine flexible Welle, die durch einen Katheter hindurch verläuft, mit einem extrakorporalen Antriebsteil verbunden. Der Antriebsteil treibt die flexible Welle an, die ihrerseits den Pumpenteil antreibt. Dabei kann der Antriebsteil wegen seines extrakorporalen Betriebs beliebig groß ausgebildet sein. Zur Herabsetzung der Reibung zwischen Welle und Katheter ist eine kontinuierliche Schmierung mit Flüssigkeit erforderlich. Ein Teil dieser Flüssigkeit gelangt durch die Gleitlager und Dichtung der Pumpeneinheit samt Abrieb in den Blutstrom. Der andere Teil wird nach der Passage durch den Katheter entlang der Welle extrakorporal gesammelt. Ferner schränkt die flexible Welle die Einsatzmöglichkeiten der Blutpumpe ein, weil diese nur zu solchen Stellen im Körper gebracht werden kann, bei denen keine allzu starken Biegungen des Katheters und der darin enthaltenen Welle erforderlich sind.

Aus W094/09835 ist eine Blutpumpe für die vorübergehende Herzunterstützung bekannt. Diese Blutpumpe, die am operativ geöffneten Herzen eingesetzt wird, weist ein stabförmiges Gehäuse auf, das den Motor und die Pumpe enthält und mit seinem Pumpenteil in die Aorta eingesetzt werden kann, während der Motorteil außerhalb der Aorta verbleibt.

Ferner ist durch EP 0 157 859 B1 eine Blutpumpe bekannt, bei der der Motorteil und der Pumpenteil baulich vereinigt sind. Diese Pumpe ist implantierbar, jedoch handelt es sich nicht um eine intravasale Blutpumpe, die minimalinvasiv in den Körper eingeführt werden kann.

Der Erfindung liegt die Aufgabe zugrunde, eine intravasale Blutpumpe zu schaffen, also eine Blutpumpe, die durch Blutgefäße hindurch vorgeschoben werden kann, welche bei der erforderlichen großen Pumpenleistung kleinformatig ausgebildet ist, und die einfach und sicher abgedichtet ist.

Eine weitere Aufgabe der Erfindung besteht darin, eine intravasale Blutpumpe zu schaffen, bei der die Gefahr der Blutschädigung durch mechanische Scherbelastung des Blutes weitgehend verringert ist.

Die erfindungsgemäße Blutpumpe ist durch den Patentanspruch 1 definiert.

Bei der erfindungsgemäßen Blutpumpe sind Antriebsteil und Pumpenteil unmittelbar miteinander verbunden und die Blutpumpe ist als stabförmiges dünnes Teil ausgeführt, wobei Motorgehäuse und Pumpengehäuse im wesentlichen gleiche Außendurchmesser haben. Der Durchmesser einer minimalinvasiv plazierbaren Blutpumpe ist auf etwa 5 bis 7 mm beschränkt, da die Gefäßweite in den Körperrandbereichen maximal etwas mehr als 7 mm beträgt. Mit einer derartigen Blutpumpe kann eine Pumpenleistung von ca. 4 l/min. bei etwa 100 mmHg Gegendruck erzeugt werden.

Erfindungsgemäß ist bei einer intravasalen Blutpumpe das Flügelrad an einem außerhalb des Motorgehäuses angeordneten Stützlager axial abgestützt und mit der Motorwelle über eine Magnetkupplung durch eine Stirnwand des Motorgehäuses hindurch gekoppelt. Die Stirnwand ist fester Bestandteil des Motorgehäuses und dichtet dieses ab. Es ist daher nicht erforderlich, eine rotierende Welle durch das Motorgehäuse hindurchzuführen. Damit entfallen an dieser Stelle Dichtungen, die die Gefahr hervorrufen, daß sie undicht werden, oder daß sich an ihnen Blut festsetzt. Die Stirnwand des Motorgehäuses besteht aus amagnetischem Material, insbesondere aus Kunststoff oder Keramik. Es besteht die Möglichkeit, das Flügelrad an einem Schaft zu lagern, der nicht rotierend ist und durch die Stirnwand des Motorgehäuses hindurchgeht. Die Abdichtung zwischen Schaft und Stirnwand ist unproblematisch, da beide Teile stationär sind.

Nach der Erfindung sind ferner Ausrichtmittel vorgesehen, um das Flügelrad in koaxialer Ausrichtung mit der Achse des Motors zu halten. Das Flügelrad ist zunächst in bezug auf das Gehäuse mit einem Stützlager axial abgestützt und radial zentriert. Ein solches Stützlager, das längs der Achse des Flügelrades angeordnet ist, verhindert aber nicht, daß das Flügelrad Pendelbewegungen um das Stützlager herum ausführt. Zur Restriktion oder Vermeidung solcher Pendelbewegungen sind entsprechende Ausrichtmittel vorgesehen, die entweder mechanischer, magnetischer oder hydromechanischer Art sein können und bewirken, daß das Flügelrad seine axiale Ausrichtung exakt beibehält. Die zentrische Anordnung des Flügelrades in bezug auf das Pumpengehäuse wird durch das Stützlager bewirkt. Die radiale Ausrichtung in bezug auf die Längsachse wird durch die Ausrichtmittel bewirkt, die separat und im Abstand von dem Stützlager vorgesehen sind.

Vorzugsweise erstreckt sich der das Flügelrad abstützende Schaft in das Motorgehäuse hinein, so daß er eine gute Führung und Konstanz seiner axialen Ausrichtung hat. Dies ist wichtig für eine schlagfreie zentrisch genaue Führung des Flügelrades. Eine derartig genaue Führung ist erforderlich, da zur Minimierung der Blutschädigung und zur Vermeidung hydraulischer Leistungsverluste der Spalt zwischen den Flügeln des Flügelrades und dem Pumpengehäuse ein Zehntel Millimeter nicht übersteigen sollte. Unrundheiten der Flügelraddrehung würden ebenfalls die Gefahr der Hämolyse vergrößern.

Ein weiterer Vorteil der Blutpumpe besteht darin, daß das Flügelrad leicht abgenommen und ausgetauscht werden kann. Das Motorgehäuse und das Pumpengehäuse bilden eine Einheit, auf die das Flügelrad als separates Teil auswechselbar aufgesteckt ist, bis es gegen das Stützlager stößt. Die aus dem Antriebsteil und dem Pumpengehäuse bestehende Einheit kann leicht gereinigt und desinfiziert werden, da sie keine Wellendurchgänge oder bewegte Teile aufweist und keine Zwischenräume vorhanden sind, in denen sich Kontaminationen festsetzen können.

Bei der Rotation des Flügelrades fördert das Flügelrad Blut in der vorgesehenen Strömungsrichtung. Dabei wirkt auf das Flügelrad eine Kraft, die bestrebt ist, das Flügelrad von dem Antriebsteil abzuziehen. Die Magnetkupplung, durch die das Flügelrad mit der Motorwelle gekoppelt ist, zieht das Flügelrad gegen das Stützlager und verhindert, daß das Flügelrad während des Betriebes von dem Stützlager abgezogen wird.

Die erfindungsgemäße intravasale Blutpumpe ist wegen ihrer guten Beweglichkeit im Gefäßsystem vielseitig einsetzbar, beispielsweise
a) als Linksherzunterstützungspumpe mit der Option (siehe b)) zur Erzeugung einer pulsierenden Strömung,
b) als Rechtsherzunterstützungspumpe mit der Option des pulsatilen Betriebs durch Modulation der Pumpendrehzahl,
c) als uni- oder biventrikuläres Unterstützungssystem während thorakaler/transthorakaler Operationen am schlagenden oder nicht schlagenden Herzen ohne den Einsatz einer Herz-Lungen-Maschine,
d) als Blutpumpe zur lokalen Organperfusion mit entsprechender Dichteinrichtung.

Im folgenden werden unter Bezugnahme auf die Zeichnungen Ausführungsbeispiele der Erfindung näher erläutert.

Es zeigen:
- Fig. 1: eine schematische Darstellung der Einführung der Blutpumpe vor den linken Ventrikel, mit Positionierung der Ansaugkanüle im linken Ventrikel,
- Fig. 2: einen schematischen Längsschnitt eines ersten Ausführungsbeispiels der Blutpumpe,
- Fig. 3: einen Längsschnitt durch ein zweites Ausführungsbeispiel der Blutpumpe,
- Fig. 4: einen Längsschnitt durch ein drittes Ausführungsbeispiel der Blutpumpe,
- Fig. 5: einen Längsschnitt durch ein viertes Ausführungsbeispiel der Blutpumpe und
- Fig. 6: eine Frontansicht des Flügelrades bei einer abgewandelten weiteren Ausführungsform.

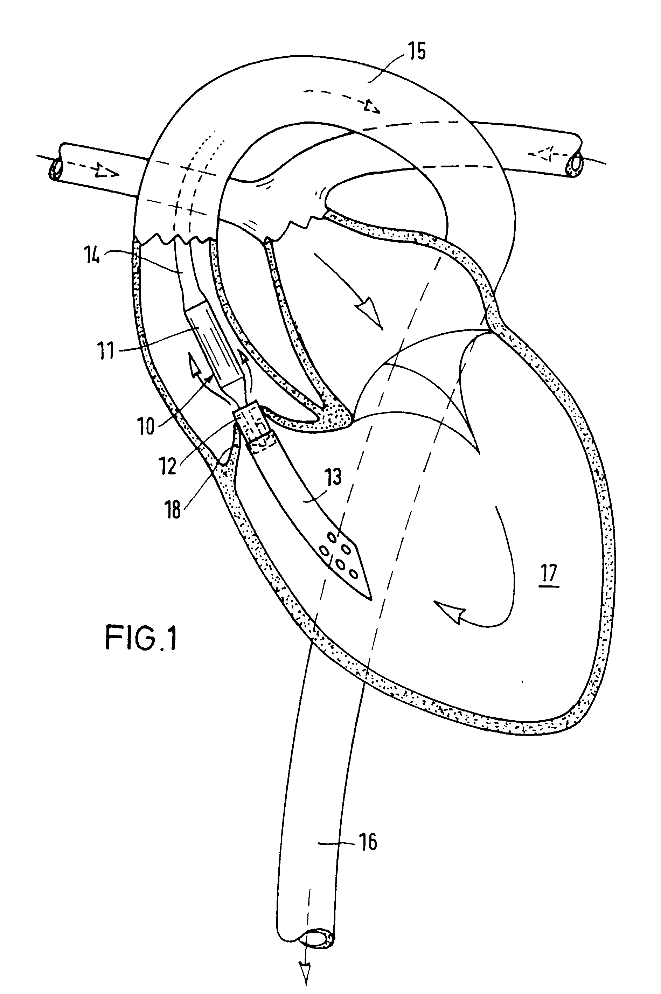

In Fig. 1 ist eine Verwendung der Blutpumpe 10 zur Linksherzunterstützung dargestellt. Die Blutpumpe 10 weist einen Motorteil 11 und einen Pumpenteil 12 auf, die koaxial hintereinander angeordnet sind und eine stabförmige Bauform ergeben. Der Pumpenteil ist durch einen Ansaugschlauch 13 verlängert, der an seinem Ende und/oder in seiner Seitenwand Öffnungen für den Blutzutritt zur Pumpe aufweist. Das dem Saugschlauch 13 abgewandte rückwärtige Ende der Blutpumpe 10 ist mit einem Katheter 14 verbunden, der durch den Aortenbogen 15 und die Aorta 16 eingeführt wurde. Die Blutpumpe 10 wird so plaziert, daß sie hauptsächlich in der aufsteigenden Aorta 16 liegt, der gerade und kurze Saugschlauch 13 aber in die Herzkammer 17 hineinragt. Die Aortenklappe 18 legt sich im Schließzustand an die Außenseite des Pumpengehäuses oder des Saugschlauchs. Die Blutpumpe 10 mit vorgelagertem Saugschlauch 13 wird durch Vorschieben des Katheters 14, ggf. mit einem darin enthaltenen Mandrin oder unter Verwendung eines Führungsdrahtes, in die dargestellte Position vorgeschoben. Der Saugschlauch 13 passiert dabei die Aortenklappe 18 retrograd, so daß durch den Saugschlauch 13 Blut angesaugt und in die Aorta 16 gepumpt wird.

Der Einsatz der erfindungsgemäßen Blutpumpe ist nicht auf die in Fig. 1 dargestellte Anwendung, bei der es sich lediglich um ein typisches Beispiel handelt, beschränkt.

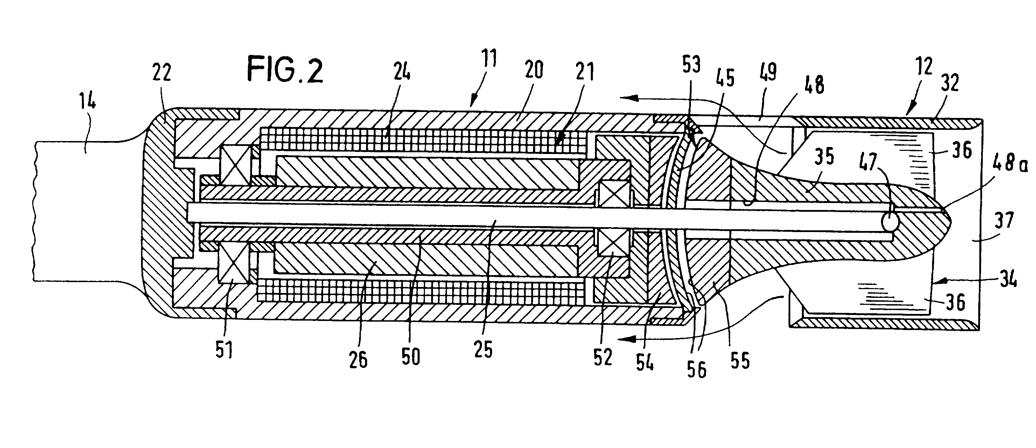

Fig. 2 zeigt ein bevorzugtes Ausführungsbeispiel der Blutpumpe mit dem Motorteil 11 und dem damit fest verbundenen Pumpenteil 12. Der Motorteil 11 weist ein langgestrecktes zylindrisches Gehäuse 20 auf, in dem der Elektromotor 21 untergebracht ist. An dem rückwärtigen Ende ist das Gehäuse 20 mit einer Stirnwand 22 verschlossen, an die sich der flexible Katheter 14 abdichtend anschließt. Durch den Katheter 14 verlaufen u.a. die elektrischen Kabel zur Stromversorgung und Steuerung des Elektromotors 21.

Der Stator 24 des Motors weist in üblicher Weise zahlreiche umfangsmäßig verteilt angeordnete Wicklungen, sowie einen magnetischen Rückschluß in Längsrichtung auf. Er ist mit dem Motorgehäuse 20 fest verbunden. Der Stator 24 umgibt einen Rotor 26, der aus in Wirkrichtung magnetisierten Permanentmagneten besteht. Der Rotor 26 ist in dem Motorgehäuse 20 drehfest angeordnet.

Der Rotor 26 des Motors sitzt auf einer mit dem Rotor 26 verbundenen Motorwelle 50, die als Hohlwelle ausgebildet ist und durch die hindurch ein Schaft 25 verläuft. Die Motorwelle 50 ist an dem rückwärtigen Ende mit einem Lager 51 im Motorgehäuse gelagert und am vorderen Ende ist sie mit einem Lager 52 auf dem Schaft 25 gelagert.

Durch das Motorgehäuse 20 erstreckt sich ein stationärer, nicht rotierender Schaft 25, der am rückwärtigen Ende mit der Stirnwand 22 verbunden ist. Der Schaft 25 ragt durch eine die Vorderseite des Motorgehäuses 20 verschließende Stirnwand 45 hindurch, in der er befestigt und stationär abgedichtet ist. Der vordere Abschnitt des Schafts 25 ragt in das Pumpengehäuse 32 hinein. Auf diesen Abschnitt ist die Nabe 35 des Flügelrades 34 aufgesetzt, wobei diese Nabe 35 sich mit einem Stützlager 47 in Form einer Kugel gegen das Schaftende abstützt. Das Stützlager 47 ist ein kombiniertes Axial-/Radiallager. Die Nabe 35 enthält eine Bohrung 48 mit Übermaß, durch die der Schaft 25 mit radialem Spiel hindurchragt, so daß die Nabe 35 leichte Pendelbewegungen um das Stützlager 47 herum ausführen kann. Durch die Nabe 35 erstreckt sich ferner eine Spülbohrung 48a, die von dem vorderen Ende der Nabe bis zu der Bohrung 48 reicht, um zur Vermeidung von Thrombenbildung eine ständige Durchströmung der Bohrung 48 zu bewirken. Das Pumpengehäuse 32 ist durch längslaufende Stege 49 mit dem Motorgehäuse 20 verbunden.

Von der Nabe 35 des Flügelrades 34 stehen Flügel 36 oder Pumpenschaufeln radial ab. Bei einer Rotation des Flügelrades 34 wird Blut durch die stirnseitige Ansaugöffnung 37 des Pumpengehäuses 32 angesaugt und in axialer Richtung im Pumpengehäuse 32 nach hinten getrieben. Durch den ringförmigen Spalt zwischen dem Pumpengehäuse 32 und dem Motorgehäuse 20 strömt das Blut entlang der sich in Fließrichtung erweiternden Nabe 35 nach außen, um dann an dem Motorgehäuse 20 entlangzuströmen. Hierdurch wird der Abtransport der im Antrieb erzeugten Wärme sichergestellt, ohne daß es zur Blutschädigung durch zu hohe Oberflächentemperaturen (über 41 °C) auf dem Motorgehäuse 20 kommt. Motorgehäuse 20 und Pumpengehäuse 32 haben etwa den gleichen Durchmesser, jedoch kann der Außendurchmesser des Pumpengehäuses 32 etwas größer sein als derjenige des Motorgehäuses, weil das Pumpengehäuse außen nicht umströmt werden muß. Das Pumpengehäuse 32 besteht aus einem zylindrischen Rohr, das am vorderen und hinteren Ende offen ist. Es ist auch möglich, bei diesem und den nachfolgenden Beispielen, den Pumpenteil mit umgekehrter Förderrichtung zu betreiben, wobei Blut an dem Motorgehäuse entlang angesaugt wird und aus dem Ende 37 axial austritt.

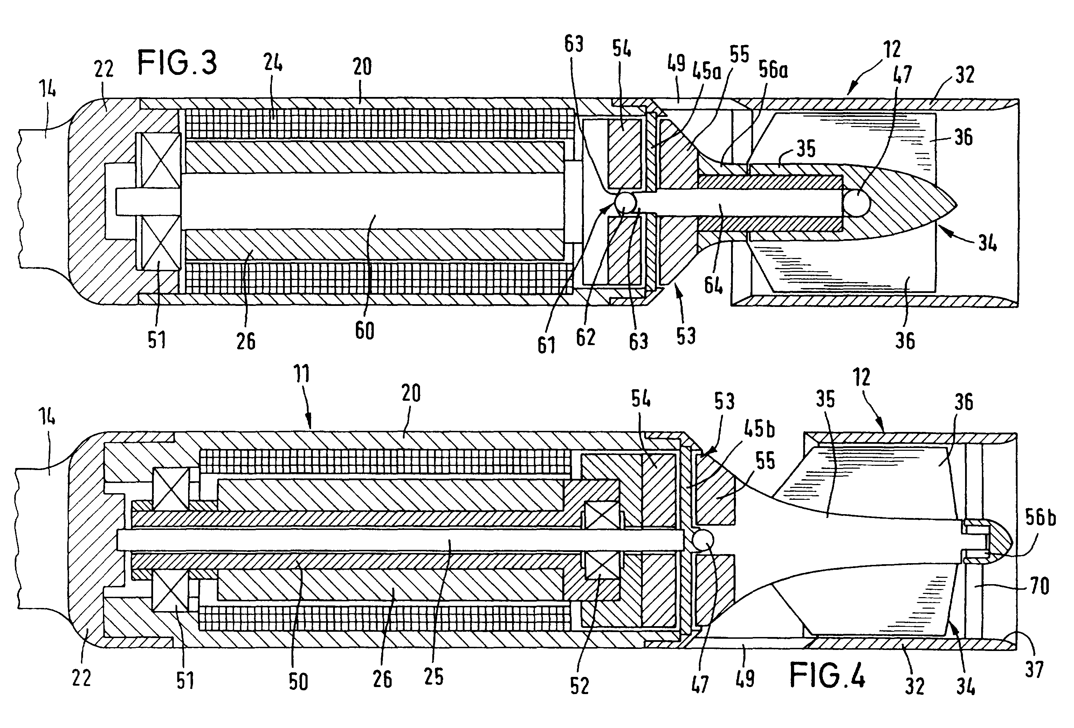

Die Drehung der Motorwelle 50 wird über eine Magnetkupplung 53 auf das Flügelrad 34 übertragen. Die Magnetkupplung weist mit der Motorwelle 50 verbundene erste Magnetteile 54 im Innern des Motorgehäuses auf sowie mit der Nabe 35 verbundene zweite Magnetteile 55. Die beiden Magnetteile sind durch die unmagnetische Stirnwand 45 magnetisch miteinander gekoppelt. Die magnetische Haltekraft der Kupplung 53 ist so stark, daß sie diejenige Kraft, die beim Pumpbetrieb bestrebt ist, das Flügelrad 34 nach vorne (in Fig. 4 nach rechts) zu treiben, überwindet, so daß das Flügelrad 34 mit der Nabe 35 ausschließlich durch magnetische Haltekraft an dem Schaft 25 festgehalten wird.

Die Stirnwand 45 ist ebenso wie die angrenzenden Wände der Magnetteile 54 und 55 zum Innern des Motorgehäuses hin konkav gewölbt, wobei das Stützlager 47 den Mittelpunkt der Wölbung bildet. Durch die Wölbung und durch die in axialer Richtung fluchtend angeordneten Magnetteile der Kupplung wird zusammen mit der Haltekraft der Magnetkupplung 53 eine Radialstabilisierung der pendelnd an dem Stützlager 47 aufgehängten Nabe durch Flüssigkeitsdämpfung erreicht. Die Wölbungen der Stirnwand 45 und der benachbarten Wand des Flügelrades 34 bilden ein Ausrichtmittel 56, um das Flügelrad beim Pumpbetrieb in koaxialer Ausrichtung zu dem Schaft 25 zu halten.

Bei dem Ausführungsbeispiel von Fig. 3 ist das Motorgehäuse 20 an seinem dem Flügelrad 34 zugewandten Ende mit einer ebenen Stirnwand 45a verschlossen. Der Rotor 26 ist auf einer Rotorwelle 60 befestigt, die am rückwärtigen Ende mit einem Kugellager 51 und an ihrem vorderen Ende mit einem Lager 61 gelagert ist. Das Lager 61 weist eine entlang der Achse der Rotorwelle 60 angeordnete Kugel 62 auf, die in einer kegelförmigen Pfanne 63 der Rotorwelle sitzt und ein Spitzenlager bildet, an dem die Rotorwelle axial abgestützt wird. Die Kugel 62 sitzt ferner in einer Lagerpfanne, die an einem Ansatz 63 ausgebildet ist, welcher von der Stirnwand 45a in das Gehäuseinnere hinein vorsteht. Das Motorgehäuse 20 ist vollständig dicht gekapselt. Zur Übertragung der Drehung der Rotorwelle 60 auf das Flügelrad 34 ist eine Magnetkupplung 53 vorgesehen, die erste Magnetteile 54 aufweist, welche mit der Rotorwelle 60 verbunden und im Innern des Motorgehäuses 20 angeordnet sind, sowie zweite Magnetteile 55, die an dem Flügelrad 34 befestigt sind. Die ersten Magnetteile 54 und die zweiten Magnetteile 55 ziehen sich durch die Stirnwand 45a hindurch an. Bei einer Drehung der Rotorwelle 60 wird auf diese Weise das Flügelrad 34 mitgedreht.

Von der Stirnwand 45a steht ein Schaft 64 nach außen ab, der in die Nabe 35 des Flügelrades 34 hineinragt und mit seinem Ende gegen eine das Stützlager 47 bildende Kugel stößt, die in der Nabe 35 befestigt ist. Zur Stabilisierung der Ausrichtung der Nabe 35 ist ein Ausrichtmittel 56a in Form eines Gleitlagers vorgesehen, das im Innern der Nabe 35 befestigt ist und um den Schaft 64 rotiert. Das Stützlager 47 bewirkt eine axiale Abstützung des Flügelrades 34 und eine hochgenaue radiale Zentrierung. Dagegen sorgt das Ausrichtmittel 56a in Form des Gleitlagers dafür, daß ein Pendeln des Flügelrades um das Stützlager 47 herum verhindert wird, und daß das Flügelrad in koaxialer Ausrichtung mit der Achse des Motors bleibt. Die Kugel des Stützlagers 47 besteht vorzugsweise aus Keramik oder einem anderen verschleißfesten Material. Sie rotiert zusammen mit dem Flügelrad.

Bei der Blutpumpe nach Fig. 4 ist der Motor generell in gleicher Weise ausgebildet wie bei Fig. 2. Durch die hohle Motorwelle 50 erstreckt sich ein Schaft 25, der in der rückwärtigen Stirnwand 22 und in der vorderen Stirnwand 45b zentriert und befestigt ist. Die Motorwelle 50 ist am rückwärtigen Ende mit einem Lager 51 im Motorgehäuse 20 und am vorderen Ende mit einem Lager 52 auf dem Schaft 25 gelagert. An der Motorwelle 50 sind die ersten Magnete 54 einer Magnetkupplung 53 befestigt. Die zweiten Magnete 55 der Magnetkupplung sind an der Nabe 35 des Flügelrades 34 befestigt.

Das Stützlager 47 besteht aus einer Kugel, die zentrisch zwischen den zweiten Magneten 55 angeordnet und an der Stirnwand 45b des Motorgehäuses 20 gelagert ist. Im Gegensatz zu den vorangehenden Ausführungsbeispielen ist in Fig. 4 das Stützlager 47 an dem motorseitigen Ende des Flügelrades 34 angeordnet, so daß kein von der Stirnwand 45b abstehender langer Stützarm erforderlich ist.

Gemäß Fig. 4 besteht das Ausrichtmittel aus einem Lager 56b, welches das stromaufwärtige Ende der Nabe 35 des Flügelrades 34 in dem Pumpengehäuse 32 lagert. Zu diesem Zwecke ist nahe der Einströmöffnung 37 ein Armstern 70 im Pumpengehäuse 32 vorgesehen, welcher das Ausrichtmittel 56b trägt. Das Flügelrad 34 ist somit an seinen beiden Enden mechanisch gelagert.

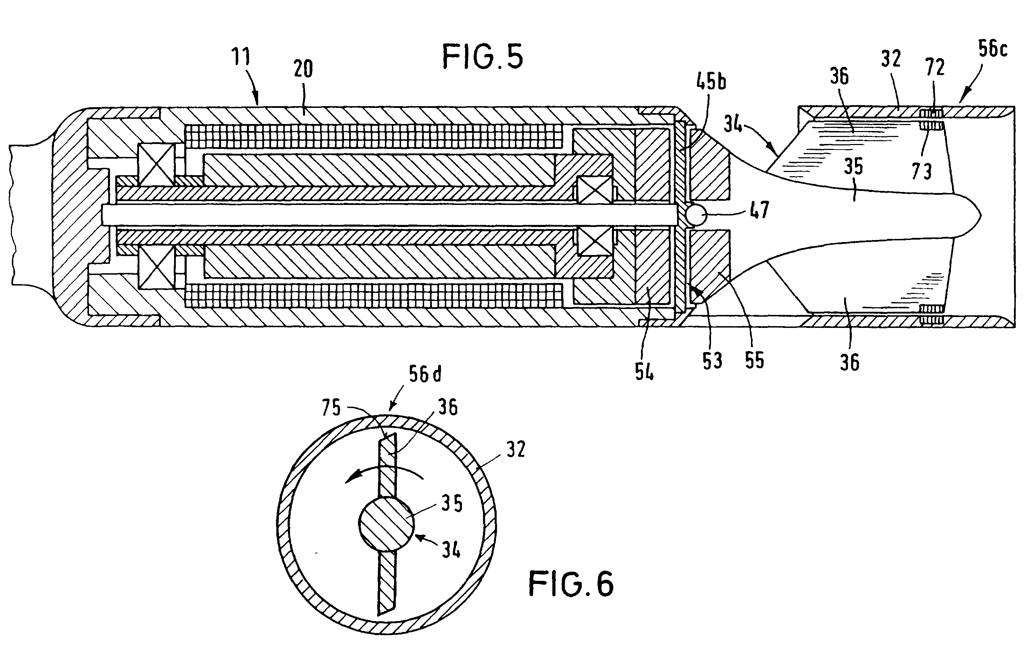

Bei dem Ausführungsbeispiel von Fig. 5 ist der Antriebsteil 11 in gleicher Weise ausgebildet wie bei Fig. 4. Auch das Stützlager 47 ist in der gleichen Weise nahe der Stirnwand 45b des Motorgehäuses 20 angeordnet. Unterschiedlich ist das Ausrichtmittel 56c. Dieses besteht in Fig. 5 aus Magneten 72,73, von denen die Magnete 72 in der Wand des Pumpengehäuses 32 und die Magnete 73 in den Flügeln 36 des Flügelrades 34 angeordnet sind. Die Magnete sind derart angeordnet, daß ihre gleichnamigen Pole einander zugewandt sind und sich gegenseitig abstoßen. Auf diese Weise wird das Flügelrad 34 im Innern des Pumpengehäuses zentriert gehalten.

Fig. 6 zeigt eine andere Ausführungsform des Ausrichtmittels 56d. Das Ausrichtmittel besteht hierbei aus Abschrägungen 75, die an den äußeren Enden der Flügel 36 des Flügelrades 34 vorgesehen sind, wobei sich der Abstand des Flügels 36 von der Umfangswand des Pumpengehäuses 32 entgegen der Drehrichtung verringert. Dies bedeutet, daß zwischen der Schrägfläche 75 und der Umfangswand des Pumpengehäuses 32 ein Druckstau entsteht, der radial nach innen auf den Flügel 36 wirkt. Auf diese Weise erfolgt eine hydrodynamische Zentrierung des Flügelrades 34.

## Patentansprüche

1. Intravasale Blutpumpe mit einem Antriebsteil (11), der in einem Motorgehäuse (20) einen Elektromotor (21) enthält und mit einem Katheter (14) verbunden ist, und einem Pumpenteil (12), der ein in einem rohrförmigen Pumpengehäuse (32) drehbares Flügelrad (34) enthält, wobei das Motorgehäuse (20) und das Pumpengehäuse (32) im wesentlichen gleichen Durchmesser haben und koaxial in axialem Abstand voneinander angeordnet sind,
**dadurch gekennzeichnet,**
daß das Pumpengehäuse (32) an dem dem Motorgehäuse (20) abgewandten Ende eine stirnseitige Ansaugöffnung (37) aufweist, daß das Flügelrad (34) an einem außerhalb des Motorgehäuses (20) angeordneten Stützlager (47) axial abgestützt ist und mit der Motorwelle (50) über eine Magnetkupplung (53) durch eine Stirnwand (45) des Motorgehäuses (20) hindurch gekoppelt ist, und daß ein Ausrichtmittel (56) vorgesehen ist, welches das Flügelrad (34) in koaxialer Ausrichtung mit der Achse des Pumpengehäuses (32) hält.

2. Blutpumpe nach Anspruch 1, dadurch gekennzeichnet, daß von der Stirnwand (45,45a) des Motorgehäuses (20) ein Schaft (25,64) absteht, der in eine Axialbohrung (48) des Flügelrades (34) ragt und das Stützlager (47) trägt.

3. Blutpumpe nach Anspruch 1 oder 2, dadurch gekennzeichnet, daß das Ausrichtmittel (56) darin besteht, daß die Stirnwand (45) des Motorgehäuses (20) konkav und das benachbarte Ende des Flügelrades (35) konvex ausgebildet ist, so daß an dem Flügelrad entlangströmende Flüssigkeit das um das Stützlager (47) pendelnde Flügelrad (34) zusammen mit der Haltekraft der Magnetkupplung (53) zentriert.

4. Blutpumpe nach Anspruch 2, dadurch gekennzeichnet, daß das Ausrichtmittel (56a) aus einem Radiallager besteht, mit dem das Flügelrad (34) auf dem Schaft (64) gelagert ist.

5. Blutpumpe nach Anspruch 1, dadurch gekennzeichnet, daß das Ausrichtmittel (56b) aus einem Radiallager besteht, mit dem das die Flügel (36) überragende Ende des Flügelrades gelagert ist und das an dem Pumpengehäuse (32) befestigt ist.

6. Blutpumpe nach Anspruch 1, dadurch gekennzeichnet, daß das Ausrichtmittel (56c) darin besteht, daß die Flügel (36) des Flügelrades (34) mit Magneten (73) versehen sind, die zusammen mit Gegenmagneten (72) am Pumpengehäuse (32) eine radiale Zentrierung des Flügelrades (34) bewirken.

7. Blutpumpe nach Anspruch 1, dadurch gekennzeichnet, daß das Ausrichtmittel (56d) darin besteht, daß die Enden der Flügel (36) des Flügelrades (34) derart ausgebildet sind, daß beim Rotieren des Flügelrades in dem Pumpengehäuse (32) eine hydrodynamische Zentrierung des Flügelrades (34) erfolgt.

## Claims

1. An intravascular blood pump comprising a drive unit (11) including an electric motor (21) in a motor housing (20) and being connected to a catheter (14), and a pump unit (12) including an impeller (34) arranged for rotation in a tubular pump housing (32), the motor housing (20) and the pump housing (32) having substantially the same diameter and being arranged coaxillay at an axial distance from each other,
**characterized in that**
the pump housing (32) on its end facing away from the motor housing (20) is provided with an end-side suction opening (37), that the impeller (34) is axially supported on a support bearing (47) arranged external of the motor housing (20) and is coupled to the motor shaft (50) by a magnetic coupling (53) through an end wall (45) of the motor housing (20), and that an orientation means (56) is provided to hold the impeller (34) in coaxial orientation with the axis of the pump housing (32).

2. The blood pump according to claim 1, characterized in that a rod (25,64), projecting from the end wall (45,45a) of the motor housing (20), extends into an axial bore (48) of the impeller (34) and supports the step bearing (47).

3. The blood pump according to claim 1 or 2, characterized in that the orientation means (56) is provided in that the adjacent end of the impeller (35) is of a convex shape so that the liquid flowing along the impeller in combination with the holding force of the magnetic coupling (53) is effective for centering the impeller (34) oscillating about the step bearing (47).

4. The blood pump according to claim 2, characterized in that the orientation means (56a) comprises a radial bearing provided to support the impeller (34) on the rod (64).

5. The blood pump according to claim 1, characterized in that the orientation means (56b) comprises a radial bearing which is provided to support the end of the impeller extending beyond the blades (36), and which is attached to the pump housing (32).

6. The blood pump according to claim 1, characterized in that the orientation means (56c) resides in that the blades (36) of the impeller (34) are provided with magnets (73) which together with counterpart magnets (72) on the pump housing (32) provide for a radial centering of the impeller (34).

7. The blood pump according to claim 1, characterized in that the orientation means (56d) resides in that the ends of the blades (36) of the impeller (34) are configured to provide for a hydrodynamic centering of the impeller (34) during the rotation of the impeller in the pump housing (32).

## Revendications

1. Pompe à sang intravasculaire, comportant une partie entraînement (11), qui contient dans un carter (20) un moteur électrique (21), et qui communique avec un cathéter (14), ainsi qu'une partie pompe (12), qui contient une roue à ailettes (34) pouvant tourner dans un corps de pompe tubulaire (32), le carter de moteur (20) et le corps de pompe (32) ayant pour l'essentiel des diamètres égaux, et étant disposés coaxialement l'un par rapport à l'autre, à une certaine distance axiale l'un de l'autre, caractérisée en ce que le corps de pompe (32) comporte, à son extrémité opposée au carter de moteur (20), une ouverture d'aspiration (37) situé du côté frontal; en ce que la roue à ailettes est en appui axial contre une butée tournante (47) disposée à l'extérieur du carter de moteur (20) et est couplée avec l'arbre moteur (50) par l'intermédiaire d'un accouplement magnétique (53), à travers une paroi frontale (45) du carter de moteur (20), et en ce qu'il est prévu des moyens d'orientation (56), qui maintient la roue à ailettes (34) en alignement coaxial avec l'axe du corps de pompe (32).

2. Pompe à sang selon la revendication 1, caractérisée en ce qu'un arbre (25, 64) dépasse de la paroi frontale (45, 45a) du carter de moteur (20), et s'étend dans un trou axial (48) aménagé dans la roue à ailettes (34) et qui porte la butée tournante (47).

3. Pompe à sang selon la revendication 1 ou 2, caractérisée en ce que les moyens d'orientation sont constitués par le fait que la paroi frontale (45) du carter de moteur (20) a une configuration concave, et l'extrémité voisine de la roue à ailettes (35) a une configuration convexe, de façon que le liquide qui s'écoule le long de la roue à ailettes assure, en même temps que la force de maintien de l'accouplement magnétique (53), le centrage de la roue à ailettes (34) qui effectue un mouvement pendulaire autour de la butée tournante (47).

4. Pompe à sang selon la revendication 2, caractérisée en ce que les moyens d'orientation (56a) sont constitués d'un palier radial, à l'aide duquel la roue à ailettes (34) s'appuie contre l'arbre (64).

5. Pompe à sang selon la revendication 1, caractérisée en ce que les moyens d'orientation (56b) sont constitués d'un palier radial qui soutient l'extrémité de la roue à ailettes qui dépasse des ailettes (36), et qui est fixé au corps de pompe (32).

6. Pompe à sang selon la revendication 1, caractérisée en ce que les moyens d'orientation (56c) sont constitués par le fait que les ailettes (36) de la roue à ailettes (34) sont pourvues d'aimants (73), qui en même temps que des contre-aimants (72) disposés sur le corps de pompe (32) assurent un centrage radial de la roue à ailettes (34).

7. Pompe à sang selon la revendication 1, caractérisée en ce que les moyens d'orientation (56d) sont constitués par le fait que les extrémités des ailettes (36) de la roue à ailettes (34) sont configurées de façon que la rotation de la roue à ailettes provoque dans le corps de pompe (32) un centrage hydrodynamique de la roue à ailettes (34).
